# EUROPEAN PATENT SPECIFICATION

(11) **EP 3 705 092 B1**
(45) Date of publication and mention of the grant of the patent: **14.10.2026**
(21) Application number: 20167327.4
(22) Date of filing: 27.02.2008
(51) Int. Cl.: A61F 2/24

(54) **VALVE ASSEMBLY**
VENTILANORDNUNG
ENSEMBLE SOUPAPE

(30) Priority: 27.02.2007 US 679750
(43) Date of publication of application: 09.09.2020
(62) Document number(s) of the earlier application(s) in accordance with Art. 76 EPC: 16181340.7 / 3 111 888; 14192682.4 / 2 853 238; 08780454.8 / 2 129 334
(73) Proprietor: Edwards Lifesciences Corporation, Irvine, CA 92614 (US)
(72) Inventor: ROWE, Stanton J., IRVINE, CA 92614 (US)
(74) Representative: Eisenführ Speiser

(56) References cited:
- WO-A1-00/47139
- US-A1- 2003 014 104
- US-A1- 2006 195 184
- US-A1- 2006 287 717

## Description

### FIELD

The present disclosure concerns apparatus for replacing a previously implanted prosthetic valve, such as a surgically implanted prosthetic heart valve, without removing the previously implanted valve from the body.

### BACKGROUND

Prosthetic valves, such as prosthetic heart valves, are implanted in the body to replace a failing or diseased natural valve. Should the prosthetic valve begin to fail, it also may need to be replaced with another prosthetic valve. Surgically implanted, prosthetic heart valves, such as a prosthetic aortic valve, typically are replaced about every 15 years. The current method for replacing a surgically implanted, prosthetic heart valve involves open heart surgery wherein the patient's chest is opened and the existing prosthetic valve is removed and replaced with a new prosthetic valve. As can be appreciated, this is a traumatic and high risk procedure accompanied by substantial morbidity and mortality, and in some cases, can not even be attempted due to the advanced age and/or medical condition of the patient.

Therefore, it would be preferable to replace a prosthetic heart valve with a percutaneously implanted valve that is delivered to the implantation site via the patient's vasculature and deployed within the previously implanted valve. However, because existing prosthetic heart valves can vary widely in size and shape, there are substantial difficulties associated with the development and validation of a percutaneously delivered replacement valve that is compatible with different types of existing prosthetic heart valves. More particularly, difficulties arise because a replacement valve that does not conform to the geometry of the previously implanted valve may not be able to adequately anchor to the previously implanted valve and/or form an effective seal with the previously implanted valve.

U.S. Patent Application Publication No. 2006/0287717 discloses a two-stage or component-based valve prosthesis that can be quickly and easily implanted during a surgical procedure. The prosthetic valve comprises a support structure that is deployed at a treatment site. The prosthetic valve further comprises a valve member configured to be quickly connected to the support structure. The support structure may take the form of a stent that is expanded at the site of a native valve. If desired, the native leaflets may remain and the stent may be used to hold the native valve open. In this case, the stent may be balloon expandable and configured to resist the powerful recoil force of the native leaflets. The support structure is provided with a coupling means for attachment to the valve member, thereby fixing the position of the valve member in the body. The valve member may be a nonexpandable type, or may be expandable from a compressed state to an expanded state. The system is particularly suited for rapid deployment of heart valves in a conventional open-heart surgical environment.

### SUMMARY

The present invention relates to an assembly and is defined by the appended claims. The following examples are useful for understanding the present invention.

In one example, the present disclosure concerns a percutaneously delivered adapter stent that is deployed with in a previously implanted prosthetic valve and serves as an anchor or platform for implanting a percutaneously delivered replacement valve within the previously implanted valve. The replacement valve can be any known percutaneous valve. The adapter stent can be adapted to provide a suitable mounting platform for implanting a percutaneous replacement valve in a wide range of existing surgical valves, which typically vary widely in size and shape from patient to patient. In one advantageous feature, the adapter stent increases the frictional forces between the percutaneous replacement valve and the failing surgical valve, thereby providing a more predictable orientation and securement of the percutaneous replacement valve. Hence, this technique is particularly suited for replacing a surgically implanted prosthetic heart valve, but also could be used for replacing a percutaneously implanted prosthetic valve.

The adapter stent can be delivered to the implantation site via the patient's vasculature and positioned within the previously implanted valve. The stent can then be deployed to cause the stent to expand and become anchored to the inner surface of the previously implanted valve. Subsequently, the replacement valve can be positioned within the adapter stent and deployed to cause the replacement valve to expand and become anchored to the adapter stent.

The adapter stent and the replacement valve can be mounted on the same delivery catheter for delivery to the implantation site. In one example, the adapter stent and the replacement valve can be crimped around respective first and second balloons of a double-balloon catheter. In this approach, the adapter stent is positioned in the previously implanted valve and expanded into contact with the previously implanted valve by inflating the first balloon. The catheter is then re-positioned to place the replacement valve in the deployed adapter stent, after which the valve is expanded into contact with the adapter stent by inflating the second balloon. In another implementation, the adapter stent and the replacement valve are self-expandable. The self-expandable adapter stent and valve can be mounted on a common delivery catheter adapted to retain the stent and the valve in compressed positions while they are advanced through the patient's vasculature. Using the catheter, the adapter stent and the valve can be successively positioned and deployed within the previously implanted valve.

The adapter stent can comprise an expandable frame that mounts a flexible annular sealing member. The sealing member provides a seal between the previously implanted valve and the replacement valve to prevent or at least minimize blood flow between the original and replacement valves.

The adapter stent may be configured to have a length that is greater than the length of the previously implanted valve that needs to be replaced. This allows the stent to extend over the entire inner surface of the previously implanted valve to provide sufficient surface area for anchoring the replacement valve and to ensure that the previously implanted valve does not interfere with the positioning and deployment of the replacement valve. In certain embodiments, the adapter stent, when expanded, has enlarged end portions that flare or extend radially outwardly past the adjacent ends of the previously implanted valve to assist in securing the adapter stent in place.

In one example not forming part of the present invention, a method is provided for percutaneously implanting a replacement prosthetic valve at a site occupied by a previously implanted prosthetic valve. The method includes positioning an adapter stent within the previously implanted valve, deploying the adapter stent to cause the adapter stent to become anchored to the previously implanted valve, positioning the replacement valve within the deployed adapter stent, and deploying the replacement valve to cause the replacement valve to become anchored to the adapter stent.

In another example not forming part of the present invention, a method of percutaneously implanting a replacement prosthetic valve in a patient at a site occupied by a previously implanted prosthetic valve includes advancing a catheter carrying an adapter stent through the patient's vasculature to position the adapter stent within the previously implanted valve. The catheter also carries the replacement valve. The method further includes deploying the adapter stent to cause the adapter stent to become anchored to the previously implanted valve, re-positioning the catheter to position the replacement valve within the deployed adapter stent, and deploying the replacement valve to cause the replacement valve to become anchored to the adapter stent.

In another example, an assembly is provided for percutaneous replacement of a previously implanted prosthetic valve without removal of the previously implanted valve. The assembly comprises an adapter stent comprising a frame and an annular sealing member. The adapter stent is adapted to be deployed within the previously implanted valve. The assembly also includes a percutaneous, replacement prosthetic valve comprising a frame and a flexible valve member. The valve is adapted to be deployed within the deployed adapter stent such that the sealing member provides a seal between the previously implanted valve and the replacement valve.

In yet another example, an assembly for percutaneous replacement of a previously implanted prosthetic valve comprises a percutaneous, replacement prosthetic valve comprising a frame and a flexible valve member. The assembly also includes means for anchoring and sealing the replacement valve to the previously implanted valve, said means being separately deployable within the previously implanted valve prior to deploying the replacement valve within said means.

The features and advantages of the invention will become more apparent from the following detailed description, which proceeds with reference to the accompanying figures.

### BRIEF DESCRIPTION OF THE DRAWINGS

FIG. 1 is a side elevation view of one embodiment of an assembly comprising a percutaneous prosthetic valve and an adapter stent for anchoring the prosthetic valve within a previously implanted prosthetic valve.
FIG. 2 is a perspective view of the prosthetic valve shown in FIG. 1.
FIG. 3 is a schematic side view of an example of a double-balloon catheter showing the prosthetic valve and the adapter stent of FIG. 1 crimped around respective balloons on the catheter for percutaneous delivery to an implantation site.
FIGS. 4A-4G illustrate the successive steps of an implantation procedure employing the double-balloon catheter shown in FIG. 3 for implanting the adapter stent and the prosthetic valve inside a failing surgically implanted, prosthetic valve previously implanted in the aortic orifice of a patient.
FIG. 5 is a schematic side view of one example of a delivery catheter that can be used to implant a self-expanding adapter stent and replacement valve inside a previously implanted valve.
FIG. 6 is a side elevation view of an example of an adapter stent that can be used to anchor a replacement valve within a previously implanted prosthetic valve.
FIG. 7 illustrates another embodiment of an implantable assembly for replacing a previously implanted prosthetic valve.
FIG. 8 illustrates the assembly of FIG. 7 deployed within a previously implanted surgical valve.

### DETAILED DESCRIPTION

As used herein, the singular forms "a", "an" and "the" refer to one or more than one, unless the context clearly dictates otherwise.

As used herein, the term "includes" means "comprises". For example, a device that includes or comprises A and B contains A and B but may optionally contain C or other components other than A and B. A device that includes or comprises A or B may contain A or B or A and B, and optionally one or more other components such as C.

The present disclosure concerns a percutaneously delivered adapter stent that is deployed within a previously implanted prosthetic valve and serves as an anchor or platform for implanting a percutaneously delivered replacement valve within the previously implanted valve. As used herein, the term "stent" refers generally to any luminal structure. The replacement valve can be any known percutaneous valve. The adapter stent can be advanced through the patient's vasculature and positioned within the previously implanted valve. The adapter stent can then be deployed to cause the adapter stent to expand and become anchored to the inner surface of the previously implanted valve. The replacement valve can then be positioned within the adapter stent and deployed to cause the replacement valve to expand and become anchored to the adapter stent. In one respect, the adapter stent is configured to increase the frictional forces between the replacement valve and the failing previously implanted valve, thereby providing a more predictable orientation and securement of the replacement valve. In the following description, the adapter stent and the replacement valve are shown and described in connection with replacing a previously implanted aortic valve. However, the embodiments described herein can also be used to replace prosthetic valves implanted at other locations in the heart or in other body channels having native valves, such as veins or other organs.

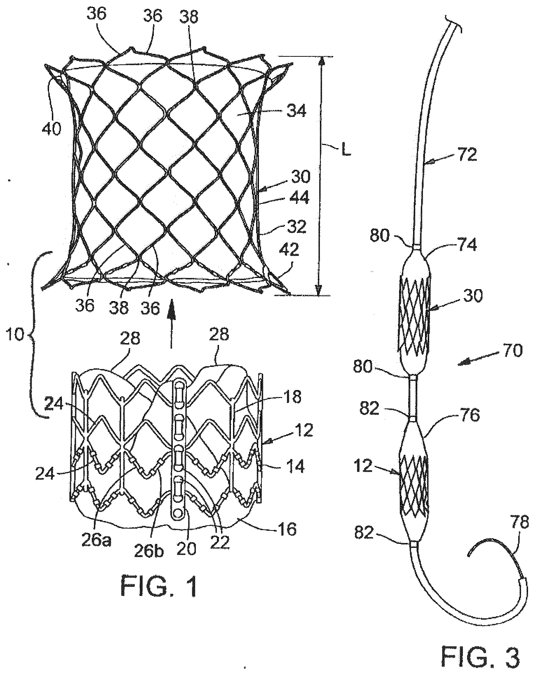

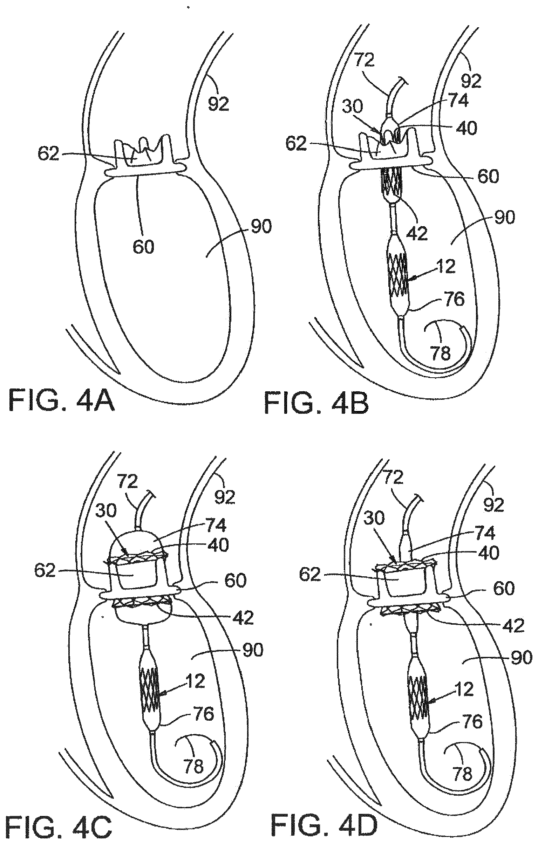

FIG. 1 shows an assembly 10 comprising a percutaneous prosthetic heart valve 12 and an adapter stent 30, according to one embodiment. The adapter stent 30 can be deployed within a failing, previously implanted valve, such as the prosthetic aortic valve 60 shown in FIG. 4A. Once the adapter stent 30 is deployed within the previously implanted valve, the new valve 12 can be deployed within the adapter stent 30 to replace the previously implanted valve 60. The previously implanted valve 60 shown in the figures is a surgical valve (i.e., a valve implanted via open heart surgery), although the adapter stent 30 and the replacement valve 12 can also be deployed within a previously implanted percutaneous valve.

The valve 12 and the adapter stent 30 are each crimpable or compressible to a reduced diameter for percutaneous delivery to the implantation site, such as using a delivery catheter. When expanded to their functional size (FIG. 1), the outer diameter of the valve 12 desirably is approximately equal to the inner diameter of the adapter stent and the outer surface of the valve 12 generally conforms to an inner surface portion of the adapter stent 30 to promote attachment of the valve 12 to the adapter stent 30. Methods for implanting the adapter stent 30 and the valve 12 are described in greater detail below, but do not form part of the present invention.

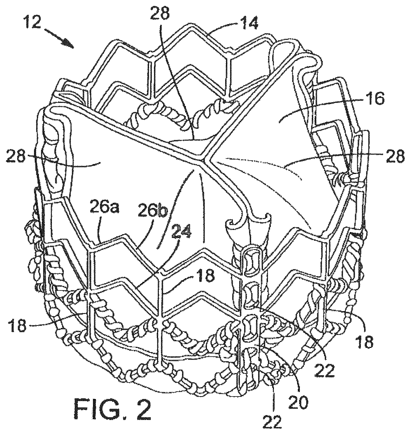

As shown in FIGS. 1 and 2, the valve 12 in the illustrated embodiment includes an annular frame 14 that mounts a flexible valve member 16. The frame 14 in the illustrated embodiment comprises a plurality of angularly-spaced axial struts, or support members, 18 that extend axially (longitudinally) along the frame and a plurality of support posts, or beams, 20 (one of which is shown in FIGS. 1 and 2) spaced in the illustrated example at 120-degree intervals from each other around the frame 14. The support posts 20 can be formed with apertures 22 to facilitate attachment of the valve member 16 to the posts 20, such as, for example, by suturing the valve member 16 to the posts. The frame 14 can also include a plurality of axially-spaced, circumferential bands, or struts, 24 attached to the axial struts 18 and the support posts 20. The struts 24 are formed with multiple bends that allow the frame 14 to be crimped to a smaller diameter for delivery to an implantation site and expanded to its functional size for anchoring the valve assembly to the adapter stent 30 at the implantation site. For example, each of the struts 24 in the illustrated configuration includes a plurality of linear strut members 26a, 26b arranged in a zig-zag or saw-tooth configuration defining bends between adjacent strut members.

In alternative embodiments, the frame 14 can have other configurations. For example, one or more of the circumferential bands 24 can have a curved or serpentine shape rather than a zig-zag shape. Further, the frame 14 can include various attachment elements (not shown), such as barbs, staples, flanges, and the like for enhancing the ability of the frame to anchor to the adapter stent 30.

The frame 14 can be made from any of various suitable ductile and/or elastic materials and is typically made of a metal, such as stainless steel, titanium, or other biocompatible metals. The frame 14 or components thereof can also be made from a shape memory alloy such as nickel titanium (NiTi) shape memory alloys, as marketed, for example, under the trade name Nitinol. The shape-memory components allow the valve 12 to be self-expandable; that is, the valve 12, when restrained in a radially compressed state by an outer restraint (e.g., a sheath covering the valve), automatically expands to its functional size when the outer restraint is removed.

The valve member 16 has a leafed-valve configuration, such as the tricuspid valve configuration shown in the illustrated embodiment. The valve member 16 can be formed from three pieces of pliant material connected to each other at seams aligned with posts 20 to form collapsible leaflets 28 (FIG. 2). The valve member 16 can be made from biological matter, such as natural tissue, pericardial tissue (such as bovine, porcine or equine pericardium), a harvested natural valve or other biological tissue. Alternatively, the valve member 16 can be made from biocompatible polymers or similar materials.

Various other prosthetic valve configurations also can be used. Examples of other valves that can be utilized are disclosed in U.S. Patent No. 6,730,118, U.S. Patent No. 6,767,362, and U.S. Patent No. 6,908,481.

The adapter stent 30 includes an expandable frame 32 that mounts a flexible annular sealing member 34. The frame 32 is shown in FIG. 1 in its expanded, functional size, and is configured to be crimpable to a reduced diameter for percutaneous delivery, such as on a delivery catheter. The frame 32 can be made from any of various suitable ductile and/or elastic materials and is typically made of a metal, such as stainless steel, titanium, or other biocompatible metals. The frame 14 or components thereof can also be made from a shape memory material, which allows the stent 30 to be self-expandable.

The frame 32 in the illustrated embodiment comprises a plurality of longitudinally extending, zig-zag struts 36 joined to each other at junctures 38. The frame 32 has a length L measured between the opposite ends thereof that desirably is greater than the length of the previously implanted valve that needs to be replaced. In this manner, the frame 32, when deployed within the previously implanted valve, can extend over the entire inner surface area of the previously implanted valve to provide sufficient surface area for anchoring the replacement valve 12 and to ensure that the previously implanted valve does not interfere with the positioning and deployment of the replacement valve 12. In particular embodiments, for example, the length L of the frame is about 10 mm to about 40 mm, with about 30 mm being a specific example.

As shown, the frame 32 has a generally cylindrical intermediate portion 44 extending between the opposite end portions 40, 42, which are enlarged or flared relative to the intermediate portion 44 when the frame is expanded. Each end portion 40, 42 desirably expands to a diameter that is greater than the diameter of the previously implanted valve. Hence, when the adapter stent 30 is deployed within the previously implanted valve, the end portions 40, 42 can extend radially outwardly past the adjacent ends of the previously implanted valve to assist in securing the adapter stent in place.

The frame 32 optionally can be provided with various attachment elements (not shown), such as barbs, staples, flanges, and the like for enhancing the ability of the frame to anchor to the previously implanted valve 60 (FIG. 4A). If desired, the frame 32 may be provided with attachment elements along the inner surface for enhancing the ability of the frame 32 to securely engage the frame 14 of the percutaneously delivered replacement valve 12.

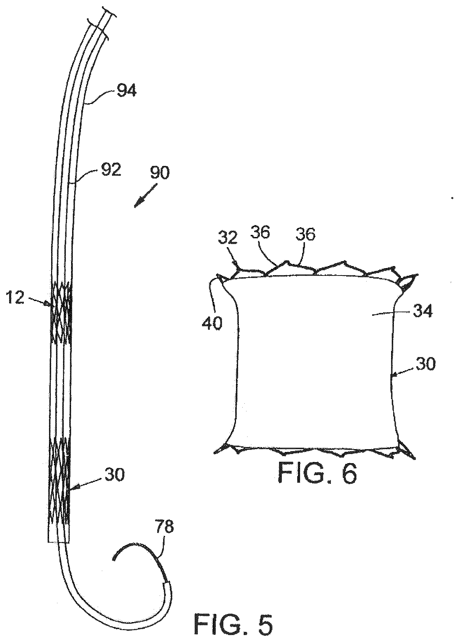

The sealing member 34 provides a seal between the previously implanted valve 60 and the replacement valve 12 to prevent or at least minimize blood flow between the valves. As shown in FIG. 1, the sealing mernber 34 desirably extends nearly the entire length of the frame 32 to maximize the surface area that can contact the previously implanted valve 60 and the replacement valve 12. Alternatively, however, the sealing member can extend along only a portion of the frame 32, such as the intermediate portion 44. With reference to FIG. 1, the sealing member 34 is secured to the inner surface of the frame 32. Alternatively, according to an example not forming part of the invention, the sealing member can be secured to the outer surface of the frame 32 as shown in FIG. 6 to prevent the leakage of blood. In another embodiment, a sealing member 34 can be secured to both the inner and outer surfaces of the frame 32.

The sealing member 34 can be made of a natural or synthetic biocompatible elastomeric material, such as foam rubber, thermoplastic elastomers (e.g., polyurethanes) or other polymeric elastomers, such as a polymeric sponge. The sealing member 34 can be secured to or formed on the frame using any suitable techniques or mechanisms, such as by suturing the sealing member to the frame or co-molding the sealing member to the frame. The sealing member 34 also can be formed on the frame using conventional coating techniques, such as spray coating, dip coating, or roll coating.

The valve 12 and the adapter stent 30 can be implanted using a double-balloon catheter. FIG. 3, for example, shows the distal end portion of an exemplary double-balloon catheter, indicated at 70. The catheter 70 includes a shaft 72, on which there are mounted first and second, spaced-apart balloons 74, 76, respectively, between a respective pair of rings 80, 82. The adapter stent 30 and the replacement valve 12 are crimped around the first balloon 74 and the second balloon 76, respectively. The shaft 72 contains two lumens (not shown), each of which is fluidly connected to a respective balloon 74, 76 for successive and separate inflation of each balloon. The shaft 72 also contains another lumen to accept a guide wire 78 so that the catheter can be advanced over the guide wire 78 for guiding the catheter through the patient's vasculature.

The catheter 70 can be introduced percutaneously into the patient's vasculature (e.g., into a peripheral artery such as the femoral artery) and advanced to the implantation site. For example, for replacing a prosthetic aortic valve, the catheter can have a length of at least about 80 cm, usually about 90-100 cm, to allow transluminal positioning of the shaft from the femoral and iliac arteries to the ascending aorta. Alternatively, the shaft may have a shorter length, e.g. about 20-60 cm, for introduction through the iliac artery, through the brachial artery, through the carotid or subclavian arteries, or through a penetration in the aorta itself. In the femoral approach, the catheter desirably is long enough and flexible enough to traverse the path through the femoral artery, iliac artery, descending aorta and aortic arch. At the same time, the catheter desirably has sufficient pushability to be advanced to the ascending aorta by pushing on the proximal end, and has sufficient axial, bending, and torsional stiffness to allow the physician to control the position of the distal end, even when the catheter is in a tortuous vascular structure. Alternatively, the catheter may be passed through a port between ribs in the patient's thorax above the heart and through an incision in the heart wall (e.g., through the apex of the left ventricle) or through an incision in the aortic arch, in a so-called minimally-invasive procedure.

An exemplary procedure for implanting the valve 12 and the adapter stent 30 using the catheter 70 is illustrated in FIGS. 4A-4G. FIG. 4A illustrates the previously implanted valve 60 implanted in the aortic annulus between the left ventricle chamber 86 and the ascending aorta 88. As noted above, the illustrated valve 60 is a surgical valve, although the adapter stent 30 and the replacement valve 12 can also be implanted within an existing percutaneous valve. The catheter 70 can be introduced percutaneously into the patient's vasculature and advanced to the implantation site using known techniques. For example, a blood vessel (e.g., the femoral artery) typically is dilated using a conventional dilator to allow an introducer sheath to be inserted into the blood vessel. The guide wire 78 can then be inserted into the blood vessel via the introducer sheath and advanced to the implantation site. Subsequently, the catheter 70 can be advanced over the guide wire 78 to position the adapter stent 30 in the previously implanted valve 60. More precisely, the adapter stent 30 desirably is positioned such that the end portions 40, 42 are located outside the adjacent ends of the previously implanted valve 60, as shown in FIG. 4B.

As depicted in FIG. 4C, the balloon 74 is then inflated to deploy the adapter stent 30, which expands to its functional size and engages the inner surface of the previously implanted valve 60. As shown, in its expanded stated, the end portion 40, 42 flare radially outwardly past the adjacent ends of the previously implanted valve to assist in retaining the adapter stent 30 in place against the valve 60. In addition, the adapter stent 30, in the illustrated example, also extends over the entire inner surface area of the existing valve 60 and causes the flexible leaflets 62 of the valve to expand radially outwardly, thereby providing a surface area suitable for mounting the replacement valve 12.

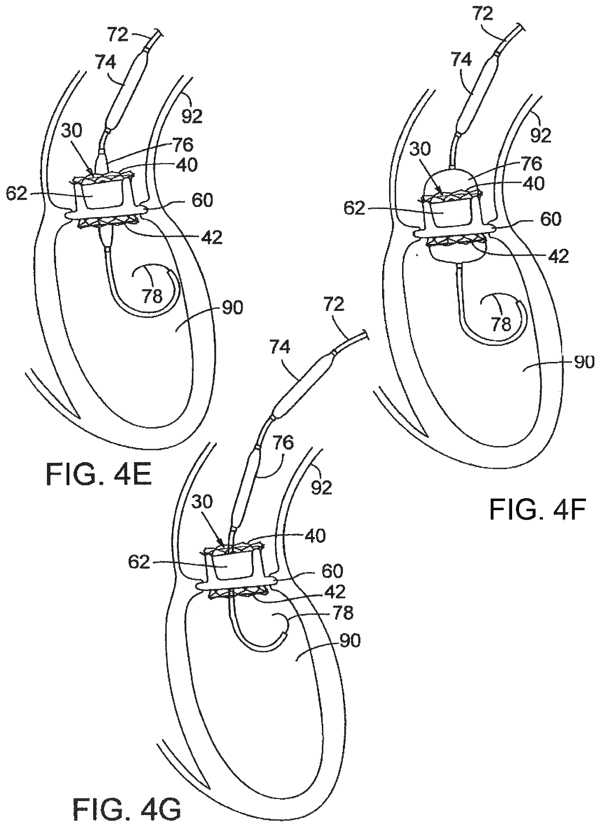

Thereafter, the balloon 74 is deflated (FIG. 4D) and the catheter 70 is retracted slightly to position the replacement valve 12 within the deployed adapter stent 30 (FIG. 4E). The second balloon 76 is then inflated to deploy the replacement valve 12, which expands to its functional size and engages the inner surface of the adapter stent 30 (FIG. 4F). Once the replacement valve 12 is deployed, the balloon 76 can be deflated and the catheter 70 can be removed from the body (FIG. 4G).

The adapter stent 30, as well as the valve 12, can be positioned at the implantation site with the assistance of fluoroscopy and radiopaque markers, ultrasonic imaging, and the like. For example, rings 80, 82 on the catheter shaft 72 can be made of any of various suitable metals that are visible during fluoroscopy for use in positioning the adapter stent and/or the valve. Alternatively, radiopaque markers can be provided on portions of the adapter stent 30 and/or the valve 12.

In an alternative approach, the replacement valve 12 can be mounted on the first balloon 74 and the adapter stent 30 can be mounted on the second balloon 76. In this approach, the adapter stent 30 is first deployed within the previously implanted valve 60 while the first balloon 74 and the replacement valve 12 are positioned in the aorta 88. After the adapter stent 30 is deployed, the catheter 70 is advanced further into the left ventricle 86 to position the first balloon 74 and the replacement valve 12 within the deployed adapter stent 30. The replacement valve 12 can then be deployed by inflating the first balloon 74.

As noted above, the frame 32 of the adapter stent 30 and the frame 14 of the replacement valve 12, or portions thereof, can be made of a shape-memory material, which allows the adapter stent 30 and the valve 12 to be self-expandable. FIG. 5 is a schematic view of the distal end portion of a delivery catheter, indicated at 90, which can be used to implant a self-expanding replacement valve and adapter stent in the previously implanted valve 60. The catheter 90 includes a shaft 92 and an outer sheath 94, which is moveable longitudinally relative to the shaft 92. The shaft 92 can include a lumen for receiving a guide wire 78. The valve 12 and the adapter stent 30 are mounted to the shaft 92 in their compressed states. The outer sheath 94 extends over the valve 12 and the adapter stent 30 to retain the valve and adapter stent in their compressed states until each is positioned for deployment at the implantation site.

The catheter 90 can be introduced into the body and advanced through the patient's vasculature in the same manner as the balloon catheter 70. The adapter stent 30 is first positioned in the previously implanted valve 60 and the outer sheath is retracted to expose the adapter stent 30, which permits the adapter stent to expand into contact with the previously implanted valve. The catheter 90 is then advanced slightly to position the valve 12 in the deployed adapter stent 30. The outer sheath 94 can then be retracted to expose the valve 12, which permits the valve to expand into contact with the adapter stent.

Although less desirable, the adapter stent 30 and the replacement valve 12 can be delivered and implanted at the site of the previously implanted valve using separate catheters. For example, the adapter stent 30 and the valve 12 can be mounted on separate balloon catheters. In this approach, the adapter stent 30 is implanted using a first balloon catheter, which is then removed from the body to allow a second balloon catheter carrying the replacement valve to be inserted into the body.

As noted above, surgical valves, such as valve 60, typically vary widely in size and shape from patient to patient. Advantageously, the adapter stent 30 can be adapted to provide a suitable mounting platform for implanting a percutaneous replacement valve in a wide range of surgical valves varying in size and shape.

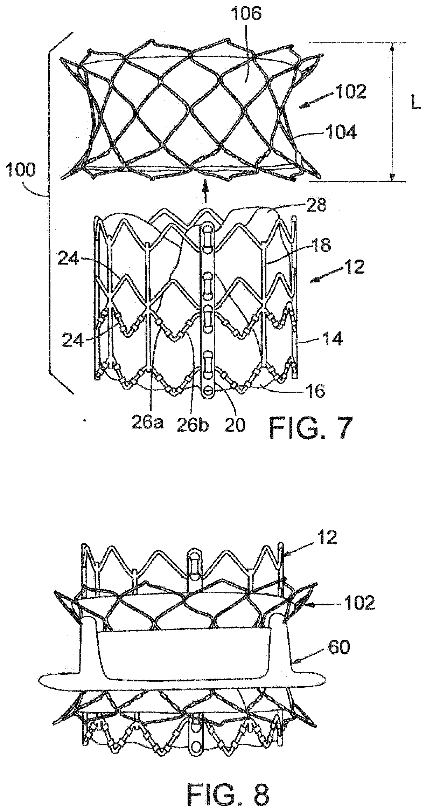

FIG. 7 illustrates another embodiment of an assembly 100 comprising a percutaneous prosthetic valve 12 and an adapter stent 102. The adapter stent 102, like adapter stent 30, includes a radially compressible and expandable frame 102 that mounts a flexible annular sealing member 106. FIG. 8 illustrates the adapter stent 102 and the prosthetic valve 12 deployed within a previously implanted surgical valve 60. The adapter stent 102 has a length L that is preferably greater than the length of the previously implanted valve 60 but need not be longer than the new valve 12. In certain embodiments, the adapter stent 102 has a length L of about 10 mm and the new valve 12 has a length of about 20 mm.

## Claims

1. An assembly (10) comprising:
adapter stent (30) comprising:
a frame (32) configured to be crimpable to a reduced diameter for percutaneous delivery, wherein the frame (32) of the adapter stent (30) is expandable to a shape having a cylindrical intermediate portion (44) extending between opposite end portions (40, 42), the end portions (40, 42) being flared relative to the intermediate portion (44) when the frame (32) of the adapter stent (30) is expanded; and
an annular sealing member (34) secured to or formed on an inner surface of the frame (32) of the adapter stent (30) and extending along at least a portion of the frame (32) of the adapter stent (30); and
a prosthetic valve (12) comprising an annular frame (14) and a flexible valve member having a leafed-valve configuration, wherein the annular frame (14) of the prosthetic valve (12) is crimpable for percutaneous delivery and expandable to a functional size, the prosthetic valve (12) being sized to be positioned within the adapter stent (30, 102).

2. The assembly of claim 1, wherein the sealing member (34) extends along the intermediate portion (44) of the frame (32) of the adapter stent (30).

3. The assembly of claim 1, wherein the sealing member (34) extends nearly the entire length of the frame (32) of the adapter stent (30).

4. The assembly of any one of claims 1 to 3, wherein the sealing member is secured to the inner surface of the frame by suturing the sealing member to the frame.

5. The assembly of any of claims 1 to 3, wherein the sealing member (34) is formed on the frame (32) of the adapter stent (30) by co-molding the sealing member (34) to the frame (32) of the adapter stent (30) or by coating the frame (32) of the adapter stent (30) by spray coating, dip coating, or roll coating.

6. The assembly of any previous claim, wherein the sealing member (34) comprises a natural or synthetic biocompatible elastomeric material.

7. The assembly of claim 6, wherein the sealing member (34) comprises a foam rubber, a thermoplastic elastomer, or other polymeric elastomer.

8. The assembly of any previous claim, wherein the adapter stent (30) comprises radiopaque markers.

9. The assembly of any previous claim, wherein the prosthetic valve (12) comprises radiopaque markers.

10. The assembly of any previous claim, wherein the flexible valve member (16) comprises three pieces of pliant material connected to each other at seams.

11. The assembly of claim 10, wherein the pliant material comprises pericardial tissue.

12. The assembly of any previous claim, wherein the adapter stent (30) has a length (L) of about 10 mm to about 40 mm, preferably about 30 mm.

13. The assembly of any previous claim, wherein the frame (32) of the adapter stent (30) and the annular frame (14) of the prosthetic valve (12) each comprise a shape-memory material.

14. The assembly of any of claims 1 to 12, wherein the frame (32) of the adapter stent (30) and the annular frame (14) of the prosthetic valve (12) each comprise a ductile metal configured to be expanded from the crimped, reduced diameter.

15. The assembly of any previous claim, wherein when the prosthetic valve (12) is expanded to the functional size, an outer diameter of the prosthetic valve (12) is approximately equal to an inner diameter of the adapter stent (30).

## Patentansprüche

1. Anordnung (10), umfassend:
einen Adapterstent (30), umfassend:
einen Rahmen (32), der ausgestaltet ist, um zur perkutanen Zufuhr auf einen reduzierten Durchmesser fältelbar zu sein, wobei der Rahmen (32) des Adapterstents (30) auf eine Form mit einem zylindrischen Zwischenabschnitt (44) expandierbar ist, der sich zwischen entgegengesetzten Endabschnitten (40, 42) erstreckt, wobei die Endabschnitte (40, 42) relativ zu dem Zwischenabschnitt (44) aufgeweitet sind, wenn der Rahmen (32) des Adapterstents (30) expandiert ist; und
ein kranzförmiges Dichtungselement (34), das an einer inneren Oberfläche des Rahmens (32) des Adapterstents (30) befestigt oder darauf gebildet ist und sich entlang mindestens eines Abschnitts des Rahmens (32) des Adapterstents (30) erstreckt; und
eine Klappenprothese (12), umfassend einen kranzförmigen Rahmen (14) und ein flexibles Klappenelement mit einer mit Segeln versehenen Klappenkonfiguration, wobei der kranzförmige Rahmen (14) der Klappenprothese (12) zur perkutanen Zufuhr fältelbar ist und auf eine funktionelle Größe expandierbar ist, wobei die Klappenprothese (12) so bemessen ist, dass sie innerhalb des Adapterstents (30, 102) positioniert ist.

2. Anordnung nach Anspruch 1, wobei das Dichtungselement (34) sich entlang des Zwischenabschnitts (44) des Rahmens (32) des Adapterstents (30) erstreckt.

3. Anordnung nach Anspruch 1, wobei das Dichtungselement (34) sich über nahezu die gesamte Länge des Rahmens (32) des Adapterstents (30) erstreckt.

4. Anordnung nach einem der Ansprüche 1 bis 3, wobei das Dichtungselement an der inneren Oberfläche des Rahmens befestigt wird, indem das Dichtungselement an den Rahmen genäht wird.

5. Anordnung nach einem der Ansprüche 1 bis 3, wobei das Dichtungselement (34) auf dem Rahmen (32) des Adapterstents (30) durch Mehrkomponenten-Spritzguss (Co-Molding) des Dichtungselements (34) an den Rahmen (32) des Adapterstents (30) oder durch Beschichten des Rahmens (32) des Adapterstents (30) mittels Sprühbeschichten, Tauchbeschichten oder Rollbeschichten gebildet wird.

6. Anordnung nach einem der vorhergehenden Ansprüche, wobei das Dichtungselement (34) ein natürliches oder synthetisches biokompatibles elastomeres Material umfasst.

7. Anordnung nach Anspruch 6, wobei das Dichtungselement (34) einen Schaumkautschuk, ein thermoplastisches Elastomer oder sonstiges polymeres Elastomer umfasst.

8. Anordnung nach einem der vorhergehenden Ansprüche, wobei der Adapterstent (30) röntgendichte Marker umfasst.

9. Anordnung nach einem der vorhergehenden Ansprüche, wobei die Klappenprothese (12) röntgendichte Marker umfasst.

10. Anordnung nach einem der vorhergehenden Ansprüche, wobei das flexible Klappenelement (16) drei Stücke eines biegsamen Materials umfasst, die an Nähten miteinander verbunden sind.

11. Verfahren nach Anspruch 10, wobei das biegsame Material perikardiales Gewebe umfasst.

12. Anordnung nach einem der vorhergehenden Ansprüche, wobei der Adapterstent (30) eine Länge (L) von etwa 10 mm bis etwa 40 mm, vorzugsweise etwa 30 mm hat.

13. Anordnung nach einem der vorhergehenden Ansprüche, wobei der Rahmen (32) des Adapterstents (30) und der kranzförmige Rahmen (14) der Klappenprothese (12) jeweils ein Formgedächtnismaterial umfassen.

14. Anordnung nach einem der Ansprüche 1 bis 12, wobei der Rahmen (32) des Adapterstents (30) und der kranzförmige Rahmen (14) der Klappenprothese (12) jeweils ein duktiles Metall umfassen, das ausgestaltet ist, um von dem gefältelten reduzierten Durchmesser expandiert zu werden.

15. Anordnung nach einem der vorhergehenden Ansprüche, wobei ein Außendurchmesser der Klappenprothese (12), wenn die Klappenprothese (12) auf die funktionelle Größe expandiert wird, annähernd gleich einem Innendurchmesser des Adapterstents (30) ist.

## Revendications

1. Ensemble (10) comprenant :
un stent adaptateur (30) comprenant :
un cadre (32) conçu pour être compressible à un diamètre réduit pour une pose percutanée, le cadre (32) du stent adaptateur (30) étant déployable en une forme présentant une partie intermédiaire cylindrique (44) s'étendant entre des parties d'extrémité opposées (40, 42), les parties d'extrémité (40, 42) étant évasées par rapport à la partie intermédiaire (44) lorsque le cadre (32) du stent adaptateur (30) est déployé ; et
un élément d'étanchéité annulaire (34) fixé à ou formé sur une surface interne du cadre (32) du stent adaptateur (30) et s'étendant le long d'au moins une partie du cadre (32) du stent adaptateur (30) ; et
une valvule prothétique (12) comprenant un cadre annulaire (14) et un élément de valvule flexible présentant une configuration de valvule à feuillets, le cadre annulaire (14) de la valvule prothétique (12) étant compressible pour une pose percutanée et déployable à une dimension fonctionnelle, la valvule prothétique (12) étant dimensionnée pour être positionnée à l'intérieur du stent adaptateur (30, 102).

2. Ensemble selon la revendication 1, l'élément d'étanchéité (34) s'étendant le long de la partie intermédiaire (44) du cadre (32) du stent adaptateur (30).

3. Ensemble selon la revendication 1, l'élément d'étanchéité (34) s'étendant quasiment sur toute la longueur du cadre (32) du stent adaptateur (30).

4. Ensemble selon l'une quelconque des revendications 1 à 3, l'élément d'étanchéité étant fixé à la surface interne du cadre par suture de l'élément d'étanchéité au cadre.

5. Ensemble selon l'une quelconque des revendications 1 à 3, l'élément d'étanchéité (34) étant formé sur le cadre (32) du stent adaptateur (30) par co-moulage de l'élément d'étanchéité (34) au cadre (32) du stent adaptateur (30) ou par revêtement du cadre (32) du stent adaptateur (30) par revêtement par pulvérisation, revêtement par immersion ou revêtement au rouleau.

6. Ensemble selon l'une quelconque revendication précédente, l'élément d'étanchéité (34) comprenant un matériau élastomère biocompatible naturel ou synthétique.

7. Ensemble selon la revendication 6, l'élément d'étanchéité (34) comprenant un caoutchouc mousse, un élastomère thermoplastique ou un autre élastomère polymère.

8. Ensemble selon l'une quelconque revendication précédente, le stent adaptateur (30) comprenant des marqueurs radio-opaques.

9. Ensemble selon l'une quelconque revendication précédente, la valvule prothétique (12) comprenant des marqueurs radio-opaques.

10. Ensemble selon l'une quelconque revendication précédente, l'élément de valvule flexible (16) comprenant trois pièces de matériau souple reliées les unes aux autres au niveau de coutures.

11. Ensemble selon la revendication 10, le matériau souple comprenant un tissu péricardique.

12. Ensemble selon l'une quelconque revendication précédente, le stent adaptateur (30) présentant une longueur (L) d'environ 10 mm à environ 40 mm, de préférence d'environ 30 mm.

13. Ensemble selon l'une quelconque revendication précédente, le cadre (32) du stent adaptateur (30) et le cadre annulaire (14) de la valvule prothétique (12) comprenant chacun un matériau à mémoire de forme.

14. Ensemble selon l'une quelconque des revendications 1 à 12, le cadre (32) du stent adaptateur (30) et le cadre annulaire (14) de la valvule prothétique (12) comprenant chacun un métal ductile conçu pour être déployé à partir du diamètre réduit comprimé.

15. Ensemble selon l'une quelconque revendication précédente, dans lequel, lorsque la valvule prothétique (12) est déployée à la dimension fonctionnelle, un diamètre externe de la valvule prothétique (12) est approximativement égal à un diamètre interne du stent adaptateur (30).
